# EUROPEAN PATENT SPECIFICATION

(11) **EP 4 759 251 B1**
(45) Date of publication and mention of the grant of the patent: **07.10.2026**
(21) Application number: 24219554.3
(22) Date of filing: 12.12.2024
(51) Int. Cl.: A61B 17/70

(54) **BONE ANCHORING DEVICE**
KNOCHENVERANKERUNGSVORRICHTUNG
DISPOSITIF D'ANCRAGE OSSEUX

(43) Date of publication of application: 17.06.2026
(73) Proprietor: Biedermann Technologies GmbH & Co. KG, 78166 Donaueschingen (DE)
(72) Inventor: BIEDERMANN, Lutz, 78048 VS-Villingen (DE); BIEDERMANN, Timo, 78647 Trossingen (DE)
(74) Representative: Prüfer & Partner mbB Patentanwälte · Rechtsanwälte

(56) References cited:
- US-A1- 2019 298 419
- US-A1- 2022 125 483
- US-A1- 2023 000 527
- US-A1- 2024 277 383

## Description

The invention relates to a bone anchoring device comprising a bone anchoring element and a receiving part for coupling a rod to the bone anchoring element. **In** particular, the bone anchoring device is a modular bone anchoring device that can be selectively adapted for various clinical requirements.

Bone anchoring devices comprising a bone anchoring element and a receiving part are used in orthopaedic surgery, in particular in spinal surgery, for coupling a rod to the bone anchoring element that is anchored in bone or a vertebra and for connecting several bone anchoring devices by the rod. **In** a polyaxial bone anchoring device, a head of the bone anchoring element is pivotably received in the receiving part, so that the receiving part can assume various angular positions in multiple planes with respect to the bone anchoring element. Thus, a suitable orientation of the bone anchoring element with respect to the rod can be achieved.

For example, a polyaxial bone anchoring device is known from US 9,345,516 B2. The polyaxial bone anchoring device includes an anchoring element having a shaft and a head, a receiving part having a first end and a second end, a bore extending from the first end towards the second end, a seat for receiving the head and at least one threaded through hole transverse to the bore. The bone anchoring device further includes a pressure element to exert pressure onto the head, such that the head is pivotable and can be locked at an angle relative to the receiving part. The pressure element has at least one hole that is at least partially aligned with the through hole. At least on set screw is threadable into the through hole to engage the pressure element at the hole such that a force is exerted by the pressure element onto the head that maintains the head at an adjustable angular position relative to the receiving part by friction.

Depending on the use or clinical application, it can be desirable to provide a bone anchoring device configured to restrict pivoting of the bone anchoring element with respect to the receiving part to a single plane, i.e. a uniplanar bone anchoring device, or to provide a monoaxial bone anchoring device in which the shank has a fixed angle, preferably a zero-angle, with respect with respect to the receiving part.

Various designs of uniplanar bone anchoring devices are known in which the shank of the bone anchoring element can pivot in the receiving part only in a single plane. For example, US 7,749,258 B2 describes a uniplanar bone anchoring device including a receiving part for receiving a rod, a pressure element and a bone anchoring element being moveable relative to the receiving part in a limited angular range about the longitudinal axis of the receiving part, the angles lying in a single plane. The movement of the bone anchoring element relative to the receiving part is limited by a form-fit connection including cooperating guiding surfaces the head of the bone anchoring element and at the pressure element.

US 2004/0204711 A1 describes a bone screw having a head and a shank. The shank has a ball thereon and the head has a socket for receiving the ball. The bone screw includes two set screws that engage the ball to lock the head in position relative to the shank.

A modular bone screw assembly is known from US 2017/0049482 A1 that includes a screw, a housing, a snap ring, a saddle, a set screw and a rod. The bone screw assembly can allow for polyaxial or uniplanar movement of the screw relative to the housing.

US 2022/0125483 A1 discloses a coupling device for connecting a rod to a bone anchor, comprising a receiving part, a pressure member, and rotatable actuating members seated in non-threaded transverse holes. Locking of the head is achieved temporarily via an eccentric cam mechanism driven by an external instrument and is fully released upon instrument retraction.

There is still a need to provide a bone anchoring device having a simple structure with only few parts that may facilitate implementation of at least a polyaxial and/or a uniplanar and/or a monoplanar bone anchoring device. Thus, it is an objection underlying the invention to provide a bone anchoring device that can be used in a modular manner enabling a selectable functionality in terms of a polyaxial, a monoaxial or a uniplanar design.

The object is solved by a bone anchoring device according to claim 1. Further developments are given in the dependent claims.

According to an embodiment of the invention a bone anchoring device for coupling a rod to bone is provided, the bone anchoring device including an anchoring element comprising a shank for anchoring in bone and a head, a receiving part having a first end and a second end, a channel for receiving the rod, a passage extending from the first end to the second end, the passage defining a central axis, and an accommodation space for accommodating the head of the bone anchoring element, and at least one through hole transverse to the central axis, the through hole comprising an internal thread. The bone anchoring device further includes at least one restriction element configured to be inserted into the through hole of the receiving part, the restriction element comprising an external thread, and a pressure element configured to be arranged in the passage and to exert pressure onto the head when the head and the pressure element are in the receiving part such that the head is pivotable with respect to the receiving part and can be locked at an angle relative to the receiving part. When the restriction element is in the through hole it is configured to interact with the pressure element or with the head to prevent a pivoting motion of the head in the receiving part or to restrict a pivoting motion of the head to a single plane.

Transverse with respect to the through hole means that an axis of the transverse hole intersects the central axis, preferably the axis of the transverse hole is substantially perpendicular to the central axis.

With the bone anchoring device, it is possible to selectively use the bone anchoring device as a polyaxial bone anchoring device or as a monoaxial bone anchoring device. The monoaxial bone anchoring device can be implemented by applying the restriction elements to prevent any pivoting motion of the head of the bone anchoring element relative to the receiving part. This can be carried out at any time before surgery. Thus, with only few parts the inventory required for surgery can be reduced.

Moreover, it is possible to use a bone anchoring device that has been originally designed as a polyaxial bone anchoring device for various monoaxial applications, such as trauma applications, growing rod applications, posterior scoliosis correction, anterior scoliosis surgery or anterior scoliosis tethering.

In a further development, the restriction elements comprise a break-off portion so that after forming the monoaxial bone anchoring device, the break-off portion can be removed to reduce an outer dimension of the bone anchoring device.

In a still further embodiment, the receiving part of the bone anchoring device is a first receiving part. A second receiving part can be coupled via the through hole to the first receiving part using a coupling element. By means of this, two rods can be coupled in parallel to a single bone anchoring element.

In a still further embodiment, the head of the bone anchoring element comprises recesses which can be engaged by restriction elements to prevent any pivoting of the bone anchoring element relative to the receiving part or to restrict a pivoting motion of the head in the receiving part to a single plane. The recesses are distinct from each other and preferably located at opposite sides of the head relative to the shank axis. Thus, by selecting a suitable restriction element either a monoaxial or a uniplanar bone anchoring device can be provided. If the restriction elements are omitted, the bone anchoring device functions as a polyaxial bone anchoring device. By the modular design, the field of applications is considerably enlarged.

In a still further embodiment, a bone anchoring device for coupling a rod to bone includes an anchoring element for anchoring in bone and a head, a first receiving part having a first end and a second end, a channel for receiving a first rod, a passage extending from the first end to the second end, the passage defining a central axis, and an accommodation space for accommodating the head of the bone anchoring element, and at least one through hole transverse to the central axis, a pressure element configured to be arranged in the passage and to exert pressure onto the head when the head and the pressure element are in the first receiving part such that the head is pivotable with respect to the receiving part and can be locked at an angle relative to the receiving part, and a second receiving part having a first end and a second end, a channel for receiving a second rod and a passage extending from the first end to a distance from the second end, the passage defining a central axis, and at least one through hole transverse to the central axis; and a connection element configured to be inserted to the through hole of the second receiving part and the through hole of the first receiving part to connect the second receiving part to the first receiving part.

With this embodiment, a bone anchoring device designed originally as a polyaxial bone anchoring device for a single rod can be easily transformed to a polyaxial bone anchoring device having two receiving parts for receiving two rods. By applying a restriction element in addition, the bone anchoring device can be transformed to a monoaxial or a uniplanar bone anchoring device.

Since the receiving part may be more expensive and/or more difficult to manufacture compared to the bone anchoring element, the manufacturing costs and/or the costs for stock holding can be decreased if the same receiving part can be used for more than one clinical application.

Further features and advantages will become apparent from the description of embodiments by means of the accompanying drawings. In the drawings:
- Fig. 1:: shows a perspective exploded view of a bone anchoring device according to a first embodiment.
- Fig. 2:: shows a perspective view of the bone anchoring device of Fig. 1 in an assembled state.
- Fig. 3:: shows a cross-sectional view of the bone anchoring device of Figs. 1 and 2, the cross-section taken in a plane extending through the central axis and perpendicular to the rod axis of an inserted rod.
- Fig. 4:: shows a perspective view from the top of a receiving part of the bone anchoring device of Figs. 1 to 3.
- Fig. 5:: shows a perspective view from the bottom of the receiving part of Fig. 4.
- Fig. 6:: shows a top view of the receiving part of Figs. 4 and 5.
- Fig. 7:: shows a cross-sectional view of the receiving part of Figs. 4 to 6 the cross-section taken along line A-A in Fig. 6.
- Fig. 8:: shows a perspective view from the top of a pressure element of the bone anchoring device of Figs 1 to 3.
- Fig. 9:: shows a perspective view from the bottom of the pressure element of Fig. 8.
- Fig. 10:: shows a top view of the pressure element of Figs. 8 and 9.
- Fig. 11:: shows a cross-sectional view of the pressure element of Figs. 8 to 10 the cross-section taken along line B-B in Fig. 10.
- Fig. 12:: shows a perspective view from the top of a restriction element of the bone anchoring device of Figs. 1 to 3.
- Fig. 13:: shows a perspective view from the bottom of the restriction element of Fig. 12.
- Fig. 14:: shows a top view of the restriction element of Figs. 12 and 13.
- Fig. 15:: shows a cross-sectional view of the restriction element of Figs. 12 to 14, the cross-section taken along line D-D in Fig. 14.
- Fig. 16:: shows a cross-sectional view of the bone anchoring device of Figs. 1 to 3 without the restriction element and with the head being pivotable in the receiving part.
- Fig. 17:: shows a cross-sectional view of the bone anchoring device of Fig. 16 with an instrument inserted into the receiving part and into the head to hold the bone anchoring element at a zero-angle position with respect to the receiving part.
- Fig. 18:: shows a cross-sectional view of the bone anchoring device of Figs. 16 and 17 with the restriction elements prior to full insertion.
- Fig. 19:: shows a cross-sectional view of the bone anchoring device of Figs. 17 and 18 with the restriction elements in an end position.
- Fig. 20:: shows a cross-sectional view of the bone anchoring device of Fig. 19 with the restriction elements in the end position and the break-off portion of the restriction elements being broken-off.
- Fig. 21:: shows a cross-sectional view of the bone anchoring device of Fig. 20 with the instrument being removed.
- Fig. 22:: shows a perspective exploded view of a second embodiment of the bone anchoring device.
- Fig. 23:: shows a cross-sectional view of the bone anchoring device of Fig. 22 in an assembled state, the cross-section taken in a plane extending through the central axis and being perpendicular to a longitudinal axis of the rod channel.
- Fig. 24:: shows a perspective view from the bottom of a second receiving part of the bone anchoring device according to Figs. 22 and 23.
- Fig. 25:: shows a perspective view from the bottom of the second receiving part of Fig. 24.
- Fig. 26:: shows a top view of the second receiving part of Figs. 24 and 25.
- Fig. 27:: shows a cross-sectional view of the second receiving part of Figs. 24 to 26, the cross-section taken along line F-F of Fig. 26.
- Fig. 28:: shows a perspective view from the top of a connection element of the bone anchoring device of Figs. 22 and 23.
- Fig. 29:: shows a perspective view from the bottom of the connection element of Fig. 28.
- Fig. 30:: shows a cross-sectional view of the connection element of Figs. 28 and 29, the cross-section taken in a plane extending through a screw axis of the connection element.
- Fig. 31:: shows a cross-sectional view of a third embodiment of the bone anchoring device, the cross-section taken in a plane extending through the central axis and being perpendicular to the longitudinal channel of the rod channel.
- Fig. 32:: shows an enlarged portion of a detail of Fig. 31.
- Fig. 33:: shows a perspective view of the bone anchoring device of Figs. 31 and 32 with inserted restriction elements.
- Fig. 34:: shows a perspective view of the bone anchoring device of Fig. 33 with the break-off portions of the restriction elements being broken-off.
- Fig. 35:: shows a perspective view from the top of the receiving part of the bone anchoring device of Figs. 31 to 34.
- Fig. 36:: shows a perspective view from the bottom of the receiving part of Fig. 35.
- Fig. 37:: shows a cross-sectional view of the receiving part of Figs. 35 and 36, the cross-section taken in a plane extending through the central axis and being perpendicular to the longitudinal axis of the rod channel.
- Fig. 38:: shows a perspective view from the top of the pressure element of the bone anchoring device of Figs. 31 to 34.
- Fig. 39:: shows a perspective view from the bottom of the pressure element of Fig. 38.
- Fig. 40:: shows a top view of the pressure element of Figs. 38 and 39.
- Fig. 41:: shows a cross-sectional view of the pressure element of Figs. 38 to 40, the cross-section taken along line G-G in Fig. 40.
- Fig. 42:: shows a perspective view of a portion of the bone anchoring element of the bone anchoring device of Figs. 31 to 34 or 46 to 49.
- Fig. 43:: shows a cross-sectional view of the bone anchoring element of Fig. 42.
- Fig. 44:: shows a perspective view from the top of the restriction element of the bone anchoring device of Figs. 31 to 34.
- Fig. 45:: shows a side view of the restriction element of Fig. 44.
- Fig. 46:: shows a cross-sectional view of a fourth embodiment of the bone anchoring device, the cross section taken in a plane extending through the central axis and being perpendicular to the longitudinal axis of the rod channel.
- Fig. 47:: shows an enlarged portion of a detail of Fig. 46.
- Fig. 48:: shows a perspective view of the bone anchoring device of Figs. 46 and 47 with inserted restriction elements.
- Fig. 49:: shows a perspective view of the bone anchoring device of Fig. 46 and 47 with the break-off portions of the restriction elements being broken-off.
- Fig. 50:: shows a perspective view from the top of the restriction element of the bone anchoring device of Figs. 46 to 49.
- Fig. 51:: shows a side view of the restriction element of Fig. 50.

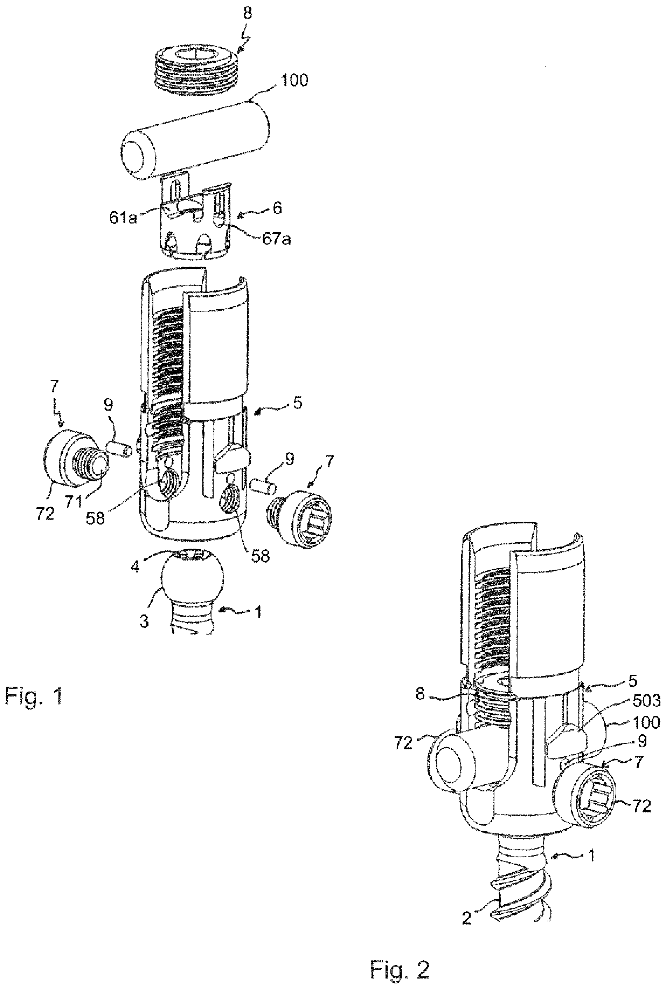

With reference to Figs. 1 to 21, a first embodiment of a bone anchoring device is described. As shown in particular in Figs. 1 to 3, the bone anchoring device includes a bone anchoring element 1 having a shank 2 for anchoring in bone or in a vertebra and a head 3. The shank 2 has a bone engagement feature, such as a thread. The head 3 is shaped as a segment of a sphere, thus comprises a spherically-shaped outer surface portion. The spherically-shaped outer surface portion preferably is smooth, i.e. is thread-free or free from corrugations. In addition, the head 3 comprises at its free end an engagement portion 4 for engagement with a tool, such as a driver. Further, the bone anchoring device includes a receiving part 5 for receiving the head 3 of the bone anchoring element 1 and for receiving a rod 100 which is configured to connect several bone anchoring devices. In the receiving part 5, a pressure element 6 is provided for exerting pressure onto the head 3 of the bone anchoring element 1 and for providing support for the rod 100. A pair of restriction elements 7 are configured to cooperate with the pressure element 6 to cause the pressure element 6 to exert such a force on an inserted head 3 that the head 3 is prevented from pivoting with respect to the receiving part 5. To lock the head 3 and the rod 100 in the receiving part 5, a locking element 8 in the form of an inner screw or set screw is provided.

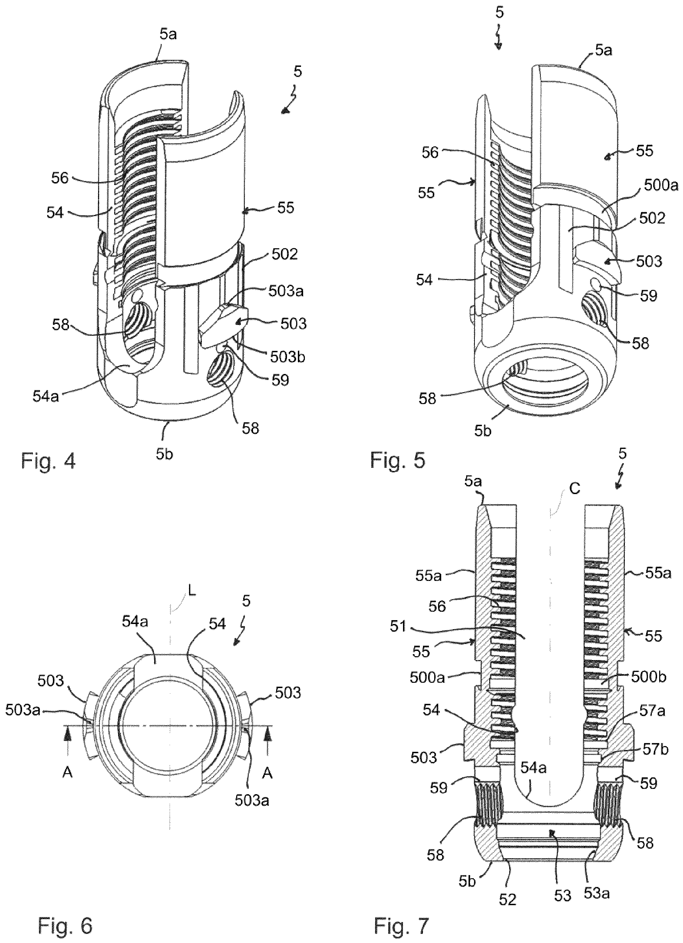

Referring in addition to Figs. 4 to 7, the receiving part will be explained in greater detail. The receiving part 5 has a first or top end 5a and a second or bottom end 5b opposite to the top end 5a. In general, the receiving part 5 may have a substantially cylindrical outer shape with a central longitudinal axis C extending through the top end 5a and the bottom end 5b. Coaxially to the central axis C, a passage 51 is provided that extends from the top end 5a to the bottom end 5b and that forms an opening 52 at the bottom end 5b. At a distance from the top end 5a, the passage 51 widens into an accommodation space 53 that is configured to receive the head 3 and at least a portion of the pressure element 6 therein. Adjacent to the opening 52 at the bottom end 5b, the accommodation space 53 narrows towards the opening 52 in a narrowing portion 53a, which may be, for example, a tapered, and more particularly a conical surface that may cooperate with a corresponding surface of the pressure element 6. The width of the opening 52 may be greater than the greatest width of the head 3, so that the head 3 may be inserted from the bottom end 5b into the accommodation space 53. To enable the insertion of the head 3 from the bottom end 5b, the width of the accommodation space 53 is such that the pressure element 6 can expand therein to permit the insertion of the head 3.

The receiving part 5 further has a substantially U-shaped recess 54 starting at the top end 5a and extending in a direction of the bottom end 5b. By means of the U-shaped recess 54 to free legs 55 are formed and define a channel that is open towards the first end 5a for receiving the rod 100.

On an inner surface of the legs 55, an inner thread 56 is formed, which is in the exemplary embodiment a square thread or another flat thread. A circumferential first groove 57a may be provided at the inner wall of the legs 55 at a distance from a bottom 54a of the U-shaped recess 54. The first inner groove 57a may provide a stop for restricting an upward movement of the pressure element 6 towards the first end 5a when the pressure element 6 is assembled with the receiving part 5 and when it is in an insertion position. Between the circumferential first groove 57a and the accommodation space 53, there may be a circumferential second groove 57b for engagement with a portion of the pressure element 6 to secure a pre-locking position of the pressure element 6.

The receiving part 5 further includes two threaded through holes 58 extending from an outer surface of the receiving part 5 into the passage 51. The threaded through holes 58 may be arranged at 180° offset from each other and at 90° with respect to the channel formed by the U-shaped recess 54. The axes a of the threaded through holes 58 extend preferably substantially perpendicular, more preferably perpendicular to the central axis C and intersect the central axis C. An axial position of the axes a of the threaded through holes 58, i.e. a distance from the bottom end 5b, is such that, when the pressure element 6 and the head 3 are inserted, the restriction elements 7 that extend through the threaded through holes 58 can engage the pressure element 6. More specifically, the position of the threaded through holes 58 is such, that a force is exerted by the restriction elements 7 via the pressure element 6 onto the inserted head 3 that prevents the head 3 from pivoting in the receiving part 5. In the embodiment, the axes a of the threaded through holes 58 are approximately at the axial position of the upper surface of an inserted head 3.

In addition, transverse holes 59, preferably with a smaller diameter than the threaded through holes 58, may extend through the legs 55, respectively, in a direction perpendicular to the central axis C and at a circumferential position approximately at the center of each of the legs 55 axially above the threaded through holes 58 in the direction towards the top end 5a. The through holes 59 may serve for accommodating pins 9, as shown in Figs. 1 to 3. The pins 9 are configured to engage the pressure element 6 to form a securing device to secure the pressure element 6 against rotation. In addition, the pins 9 may limit an upward movement of the pressure element 6.

At a distance from the bottom end 5b and above the bottom 54a of the U-shaped recess 54 a circumferential groove 500a may be provided in the outer surface of each of the legs 55. At a corresponding position on the inner surface of the legs 55 a circumferential groove 500b may be provided so that at the position of the outer groove 500a the legs 55 are weakened. As a result, an upper portion 55a of the legs 55, respectively, up to the top end 5a, may be broken-off. Thus, the upper portion 501 of the legs 55, respectively, serve as extended tabs that allow to guide instruments or the fixation screw 8 downwards on their intended place. This is particularly useful in minimally-invasive surgery (MIS), where only small incisions are made in a patient's skin.

At the outside of the legs 55, for example, longitudinal recesses 502 and/or attachment projections 503 may be provided for engagement with a tool or an instrument. The attachment projections 53 may be located above the threaded through holes 58 and the transverse holes 59 in a direction towards the top end 5a but below the grooves 500a. In greater detail, the attachment projection 503 may have in a plane view onto the projection 503 a substantially triangular shape with a tip 503a facing towards the top end 5a. A width of the attachment projection 503 at the base 503b opposite to the tip 503a in a circumferential direction may be the same as or greater than a width of the threaded through holes 58.

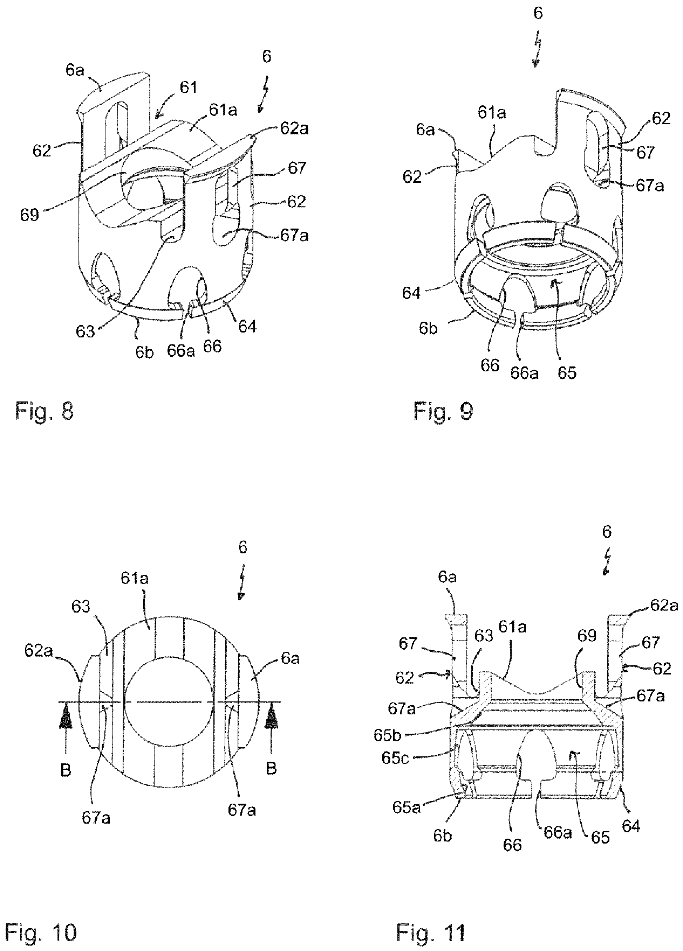

Referring further to Figs. 8 to 11, the pressure element 6 is described. Preferably, the pressure element 6 is a monolithic piece which is configured to be arranged in the passage 51 and to encompass the head 3 laterally and from a free end of the head to exert pressure onto the head when the head 3 and the pressure element 6 are in the receiving part 5 such that the head is pivotable with respect to the receiving part and can be locked at an angle relative to the receiving part.

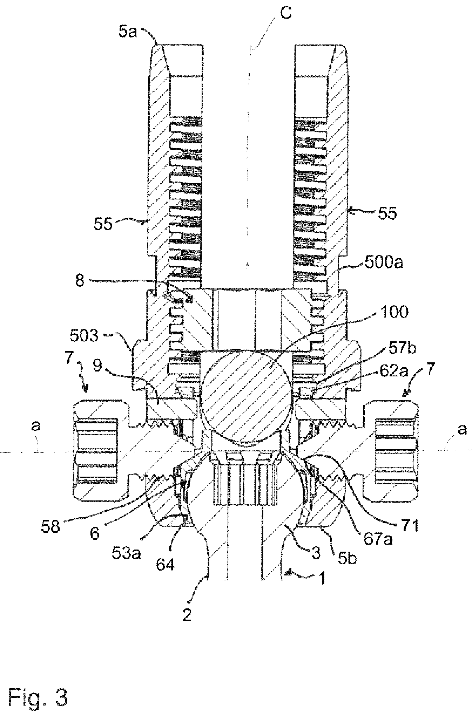

In greater detail, the pressure element 6 has a first or top end 6a and a second or bottom end 6b, and may be substantially cylindrical with an outer diameter that allows the pressure element 6 to move in the passage 51 of the receiving part 5. At the top end 6a, a rod-receiving recess 61 is formed with a rod support surface 61a. The rod support surface 61a may have a substantially V-shaped cross-section with a longitudinal axis L extending substantially perpendicular to a cylinder axis of the pressure element 6 which coincides with the central axis C of the receiving part 5 when the pressure element 6 is in the receiving part 5. The depth of the rod-receiving recess 61 may be smaller than a diameter of the rod 100. Hence, when the rod 100 rests on the rod support surface 61a, the rod projects over the top end 6a of the pressure element as shown, for example, in Fig. 3. The V-shape of the rod support surface 61a more easily facilitates use of rods with different diameters.

Moreover, the rod-receiving recess 61 is shaped such that two free legs 62 are formed that may be separated from the rod support surface 61a on each side by a groove 63. By means of this, the legs 62 are slightly flexible in a direction transverse to the longitudinal axis of the rod support surface 61. A free end of the legs 62 may have a radially protruding rim 62a, an upper surface of which forms the top end 6a of the pressure element 6. The rim 62a is configured to engage the grooves 57a, 57b provided at the inner surface of the legs 55 of the receiving part 5, to secure the insertion position or the pre-locking position of the pressure element 6 in the receiving part 5.

Adjacent to the bottom end 6b of the pressure element 6, an outer surface portion 64 may be tapered, preferably conically-shaped, which outer surface portion 64 is configured to cooperate with the narrowing portion 53a of the receiving part 5. Further, a hollow head receiving portion 65 is formed in the pressure element 6 with an opening at the bottom end 6b for inserting the head 3. The head receiving portion 65 may have a lower and an upper substantially spherical section 65a, 65b that are shaped so as to matingly receive the spherical head 3. An intermediate section 65c has a greater inner diameter for facilitating the insertion of the head 3. In addition, a plurality of recesses 66 are formed that extend completely through the pressure element 6 into the widened section 65c and which are open to the second end via substantially axially extending slits 66a. The recesses 66 may have an inverted drop shape, with the broader portion located closer to the bottom end 6b. The recesses 66 and the slits 66a are configured to spread when the head 3 is inserted. In general, the number, shape and size of the recesses 66 are selected such that a desired flexibility is achieved that allows insertion of the head 3 through the bottom end until the head 3 is received in the head-receiving portion 65. The size of the head-receiving portion 65 may also be such that the head 3 can be held therein by friction before a final locking is effected.

At the center of each of the legs 62 in a circumferential direction an axially extending elongate through hole 67 is provided, that serves for receiving the pins 9 and the restriction elements 7 as shown in Figs. 1 to 3. For receiving a portion of the restriction elements 7, the bottom of the elongate through hole 67 comprises a broadened portion with a substantially conical shape that forms a first cooperating surface 67a that is configured to cooperate with a second cooperating surface at the restriction element 7 as described in greater detail below. The first cooperating surface 67a extends transversely to the central axis C at an axial position below the groove 63 and slightly above the upper spherical portion 65b of the head-receiving portion 65 as can be seen in particular in Fig. 11. This permits to exert a force with the restriction elements 7 directly onto an upper portion of an inserted head 3 via the cooperating surfaces of the pressure element 6 and the restriction element 7. Lastly, a coaxial bore 69 is formed in the pressure element 6 for permitting access with a driver to the engagement recess 4 of the head 3.

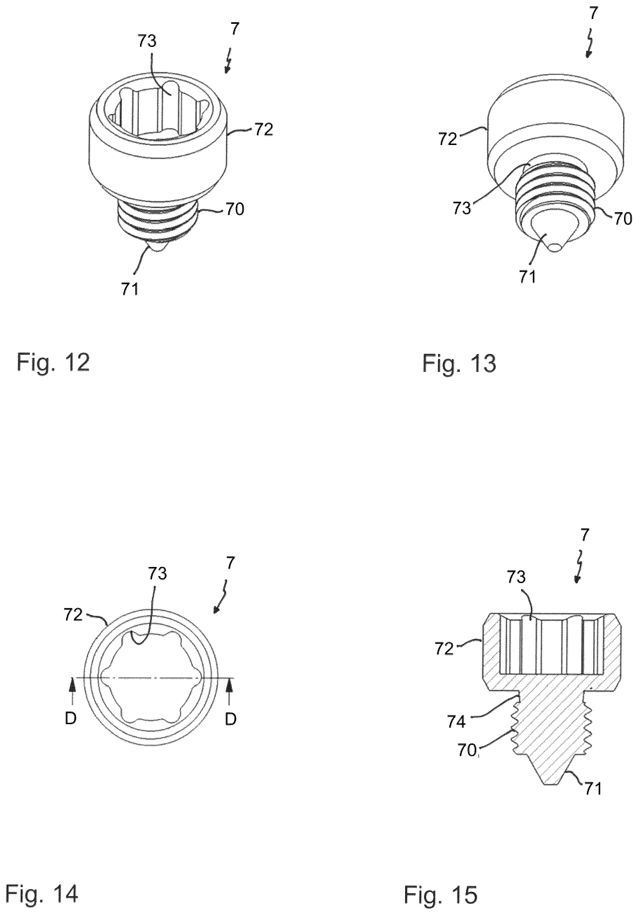

Referring now to Figs. 12 to 15, the restriction element 7 is described. The restriction element 7 comprises a threaded portion 70 that is configured to cooperate with the threaded through hole 58 of the receiving part 5. A front portion 71 is formed as a cone that projects from the threaded portion 70 and that may have a flat end portion. An outer diameter on the base of the front portion 71 adjacent to the threaded portion 70 is smaller than an outer diameter of the threaded portion 70. In greater detail, the front portion 71 has a shape such that it fits into the lower conical portion of the elongate hole 67 of the pressure element 6. Thus, an outer surface of the conical front portion 71 forms the second cooperating surface that is configured to cooperate with the first cooperating surface 67a of the pressure element 6. A rear portion 72 on the other side of the threaded portion 70 and opposite to the front portion 71 forms a head of the restriction element 7. The rear portion 72 is substantially cylindrical and has a greater outer diameter than the threaded portion 70. In the free end surface of the rear portion an engagement recess 73 for engagement with a tool, such as a driver, is provided. The engagement recess 73 may have a torx-shape as depicted in Figs. 12 and 14. The overall size of the rear portion 72 is such that it can be easily gripped so that the restriction element 7 can be screwed into the threaded hole 58 of the receiving part 5. Between the threaded portion 70 and the rear portion 72 a predetermined breaking area 74, i.e. a weakened section, is formed that has a slightly smaller outer diameter than the threaded portion 70. The rear portion 72 is configured to be broken-off a the predetermined breaking area 74 when a torque is applied to the rear portion 72 that exceeds a predetermined torque.

The parts and portions of the bone anchoring device may be made of any material, preferably, however, of titanium or stainless steel or any bio-compatible metal or metal alloy or plastic material. For bio-compatible alloys, a NiTi alloy, for example, Nitinol, may be used. Other materials that can be used are, for example, magnesium or magnesium alloys. Bio-compatible plastic materials that can be used may be, for example, polyether ether ketone (PEEK) or poly-L-lactide acid (PLLA). The parts can be made of the same or of different materials from one another.

In use, the bone anchoring device can be applied as a polyaxial bone anchoring device or as a monoaxial bone anchoring device.

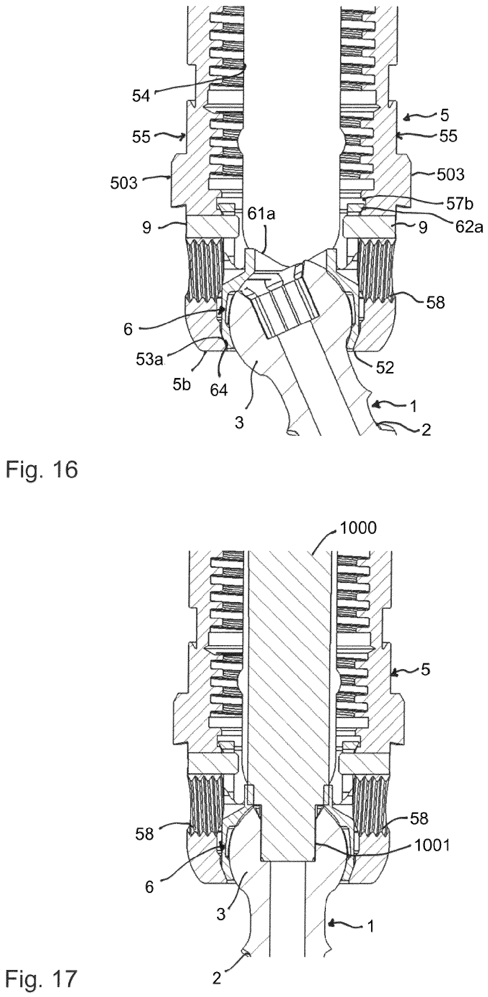

As shown in Fig. 16, the pressure element 6 is pre-assembled with the receiving part 5 such that the upper rim 62a of the pressure element 6 extends into the second groove 57b and the rod support surface 61a is aligned with the channel formed by the U-shaped recess 54. The pins 9 extend through the through holes 59 into the elongate recesses 67. Thereby, the pressure element 6 is secured against rotation. The head 3 of the bone anchoring element 1 is inserted from the bottom end 5b into the head-receiving portion 65 of the pressure element. When the outer rim 62a is held into the second groove 57b, the pressure element 6 is in the pre-locking position, in which the narrowing outer surface portion 64 at the lower end of the pressure element 6 engages the narrowing inner surface portion 53a of the receiving part 5, so that the head 3 cannot be removed through the lower opening 52. In this position, the head 3 is still pivotable in the receiving part 5 as shown in Fig. 16. When the bone anchoring device shall be used as a polyaxial bone anchoring device, the rod 100 is inserted into the U-shaped recess until it rests on the rod support surface 61a of the pressure element 6. To lock the bone anchoring device, i.e. the head 3 and the rod 100 in the receiving part 5 , the locking element 8 is screwed between the legs 55 until it presses onto the rod so that the pressure element is moved further downward until it locks the head 3 in a particular angular position with respect to the receiving part 5.

When the bone anchoring device shall be used as a monoaxial bone anchoring device, i.e. when the axis of the shank 2 is permanently coaxial with the central axis C of the receiving part 5, the restriction elements 7 are used. First, as shown in Fig. 17, an instrument 1000 that comprises an elongate bar with a front portion 1001 is inserted into the receiving part 5 from the top end 5a until the front portion 1001 engages the tool engagement recess 4 of the head 3. By means of this, the bone anchoring element 1 assumes a zero-angle position relative to the receiving part 5, i.e. the shank axis of the shank 2 is coaxial with the central axis C.

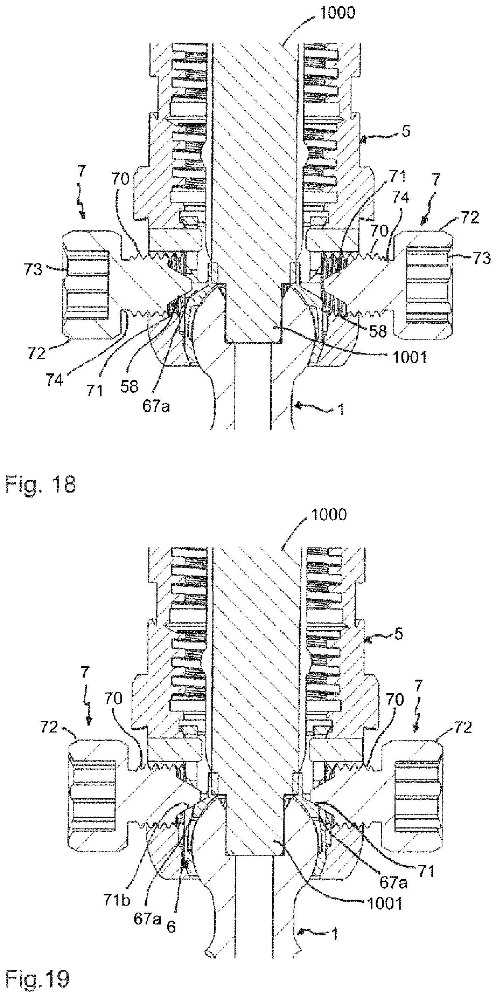

Next, as depicted in Fig. 18, the restriction elements 7 are screwed into the threaded holes 58, respectively, until the conical front portion 71 with the second cooperating surface engages the first cooperating surface 67a in the lower portion of the elongate hole 67 of the pressure element 6 as depicted in Fig. 19. By means of this, an increasing pressure is exerted via the pressure element 6 onto the head 3 until the bone anchoring element 1 is locked in the zero-angle position. The position of the longitudinal axes a of the threaded through holes 58 is selected such that the force generated by the restriction elements 7 achieve a full or almost full locking of the head 3 in the receiving part 5. Also, the bone anchoring element 1 is prevented from rotation when the restriction elements 7 are fully tightened.

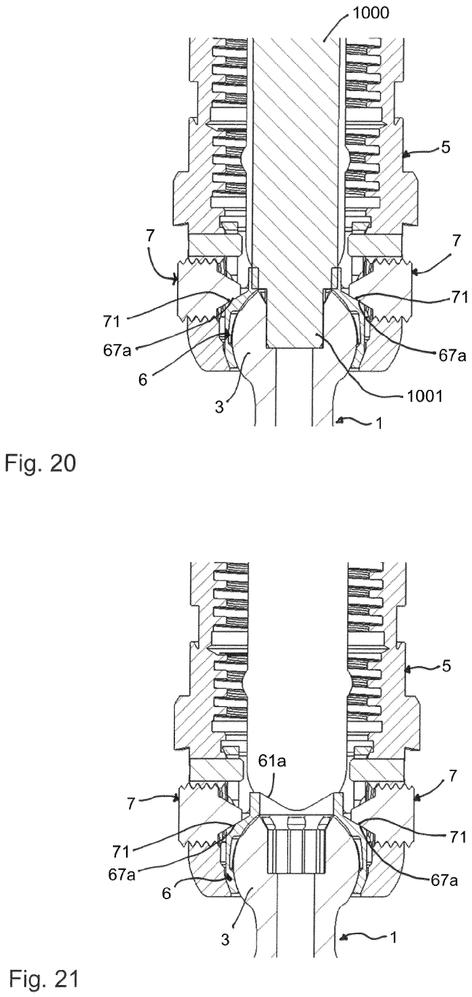

When the counterforce exerted by the first cooperating surface 67a onto the second cooperating surface 71 of the restriction element 7 exceeds a predefined value, the rear portion 72 is broken-off from the threaded portion 70 at the predetermined breaking area 74 as depicted in Fig. 20. Since the head 3 of the bone anchoring element 1 is locked in a zero-angle position, the instrument 1000 can be removed subsequently as shown in Fig. 21. Hence, in Fig. 21, a monoaxial bone anchoring device is shown that can be used like any other monoaxial bone anchoring device. By engagement of the tool recess 4 of the head with a driver that extends through the bore 69 in the pressure element, the monoaxial bone anchoring device can be inserted into a bone part or a vertebra.

It shall be noted that the break-off portions 55a of the legs 55 can be broken-off after final locking of the bone anchoring device with the locking element 8.

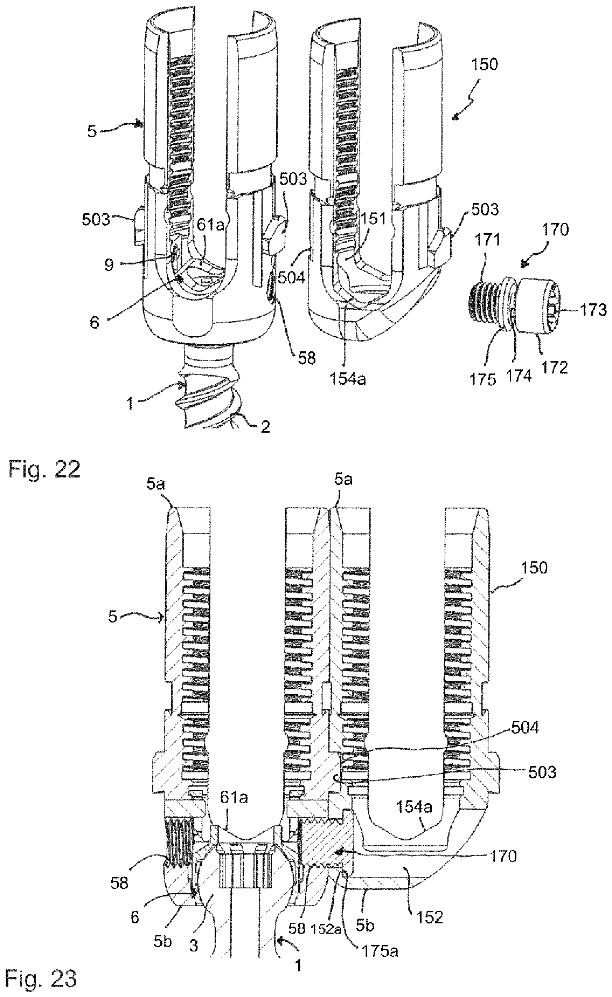

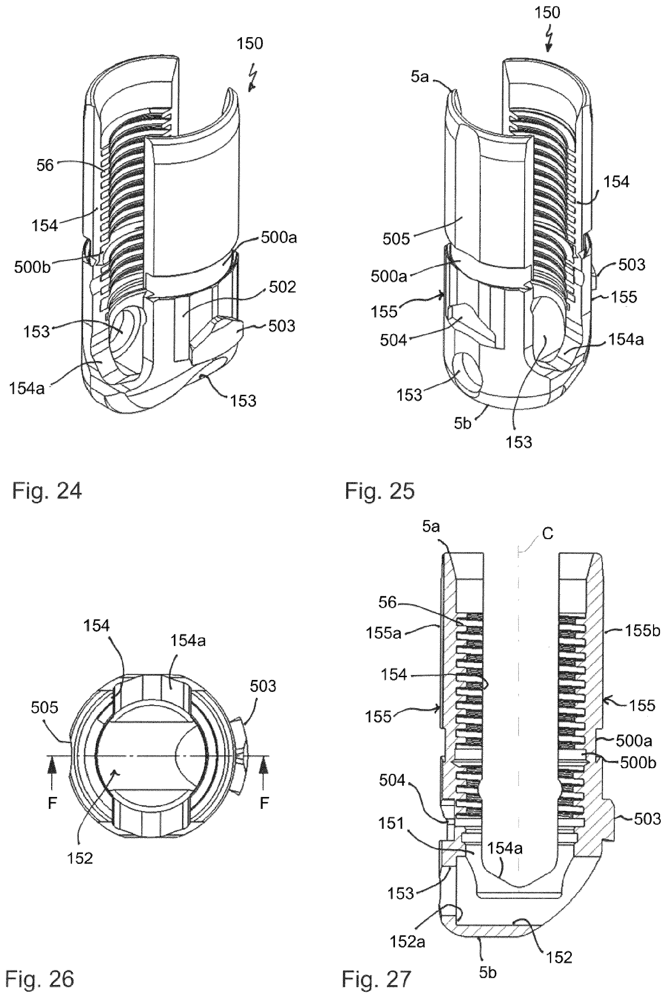

Referring to Figs. 22 to 30, a second embodiment of the bone anchoring device is described. Parts and portions that are identical or highly similar to the first embodiment, are marked with the same reference numerals and the description thereof is not repeated. As can be seen in Figs. 22 and 23, the bone anchoring device according to the second embodiment includes the bone anchoring device according to the first embodiment, i.e. the receiving part 5 with the pressure element 6 and the bone anchoring element 1. In the second embodiment, the receiving part 5 forms a first receiving part. In addition, the bone anchoring device includes a second receiving part 150 and a connection element 170. The connection element 170 is configured to connect the second receiving part 150 to the first receiving part 5 using one of the threaded through holes 58 in the first receiving part 5. By means of this, two rods can be connected to a single bone anchoring element 1. Such an application may be used, for example, in a growing rod application, in which, for example, one rod is fixed and the other rod is slidable. This may be particularly useful in the treatment of scoliosis.

The second receiving part 150 differs from the first receiving part 5 in various aspects, but mainly in that the lower portion lacks a head-receiving portion and instead has features for connecting the second receiving part 150 to the first receiving part 5. The second receiving part 150 is substantially cylindrical and comprises a passage 151 that ends at a distance from the bottom end 5b. The bottom end 5b comprises a substantially flat surface. A transverse bore 152 is formed above the bottom end 5b and at an axial position below the triangular-shaped attachment projection 503. An inner diameter of the transverse bore is greater than an outer diameter of a rear portion 172 of the connection element 170 (see Fig. 23), such that the connection element 170 can be introduced and guided in the transverse bore 152. The transverse bore 152 is open to the side where the triangular projection 503 is located. A portion of the receiving part 150 around the transverse bore 152 is cut away so that the outer surface from the bottom end 5b towards the triangular projection 503 is rounded. On the opposite side, the transverse bore 152 comprises a section 153 with a smaller diameter such that a shoulder 152a is formed between the transverse bore 152 and the section 153. The shoulder 152a forms an abutment for a portion of the connection element 170. A rod-receiving recess 154 extends from the top end to a distance from the bottom end. The rod-receiving recess 154 has a V-shaped bottom region 154a that allows to support rods of different diameter. By means of the recess 154 two free legs 155 are formed that comprise an upper portion 154a above the outer groove 500a and the inner groove 500b as in the first embodiment. The upper portion 155a may be broken-off. On the leg 155 opposite to the leg 155 with the triangular projection 503, a triangular-shaped recess 504 is formed that has a shape corresponding to the shape of the triangular projection 503 and which is located at a position complementary to the position of the triangular-shaped projection 503 of the first receiving part 5, so that the triangular projection 503 of the first receiving part 5 can be received in the triangular recess 504 of the second receiving part 150. Moreover, in the outer surface of the leg 155 that has the triangular-shaped recess 504 a shallow groove 505 is formed that extends parallel to the central axis C from the top end 5a to substantially the bottom end 5b. The shallow groove 505 has a substantially cylindrical shape so as to fit to the outer surface of the leg 55 of the first receiving part 5.

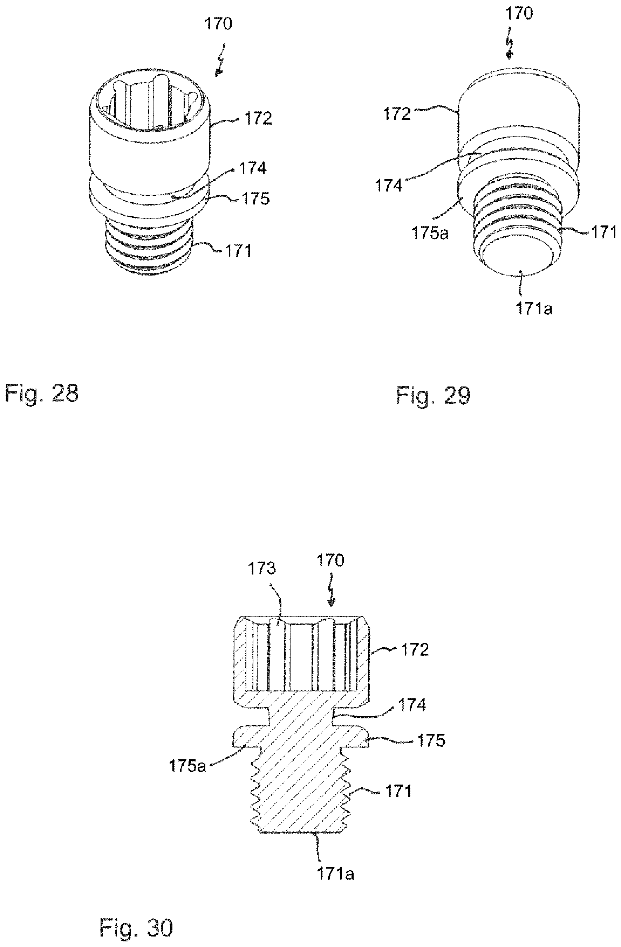

Referring in particular to Figs. 28 to 30, the connection element 170 has a threaded portion 171 that is configured to be threaded into the threaded through hole 58 of the first receiving part 5. The front end of the connection element 170 at the threaded portion may have a flat end surface 171a. A rear portion 172 may be shaped like in the restriction element 7 of the first embodiment, i.e. the rear portion 172 may have a cylindrical shape with a greater outer diameter than the outer diameter of the threaded portion 171 and a tool engagement recess 173. Adjacent to the rear portion 172, a predetermined breaking area 174 is formed and between the predetermined breaking area 174 and the threaded portion 171, a ring-shaped abutment portion 175 is provided. The abutment portion 175 has a flat abutment surface 175a that faces towards the front end 171a and that is configured to abut against the shoulder 152a of the second receiving part 150. An outer edge of the abutment portion 175 that faces towards the rear portion 172 may be rounded.

In use, as can best be seen in Figs. 22 and 23, the second receiving part 150 can be attached to the first receiving part 5, so that the bone anchoring device is a bone anchoring device for two rods. First, the first receiving part 5 and the second receiving part 150 are aligned in such a way that the two rod channels are oriented in parallel. Then, the projection 503 of the first receiving part 5 is inserted into the complementary groove 504 at the second receiving part 150. Subsequently, the connection element 170 is inserted into the transverse bore 152 until the threaded portion 171 extends through the section 153 of the transverse bore 152 into the threaded through hole 58 of the first receiving part 5. The connection element 170 is then tightened until the abutment surface 175a abuts against the shoulder 152a. When a predetermined torque is exceeded, the rear portion 172 of the connection element 170 is broken-off at the predetermined breaking area and can be removed. The first receiving part 5 and the second receiving part 150 are thus fixedly connected. Finally, the bone anchoring element 1 can be inserted into a bone or a vertebra and rods 100 can be inserted into the first receiving part 5 and into the second receiving part 150. Depending on the clinical application, either both rods can be fixed with a locking member 8 or only one of the rods can be fixed with a locking member 8 and the other rod can remain slidable in the respective receiving part. It shall be noted that the first receiving part 5 and the second receiving part 150 each can receive rods of different diameter due to the V-shape of the rod support surface 61a of the pressure element 6 and the bottom 154a of the rod-receiving recess 154.

In the embodiment shown in Fig. 23, the bone anchoring device is still polyaxial, i.e. the head 3 is pivotable in the first receiving part 5. However, by using a restriction element 7 as shown in the first embodiment, that is inserted into the transverse through hole 58 opposite to the transverse through hole 58 in which the connection element 170 has been inserted, a monoaxial bone anchoring device can also be provided.

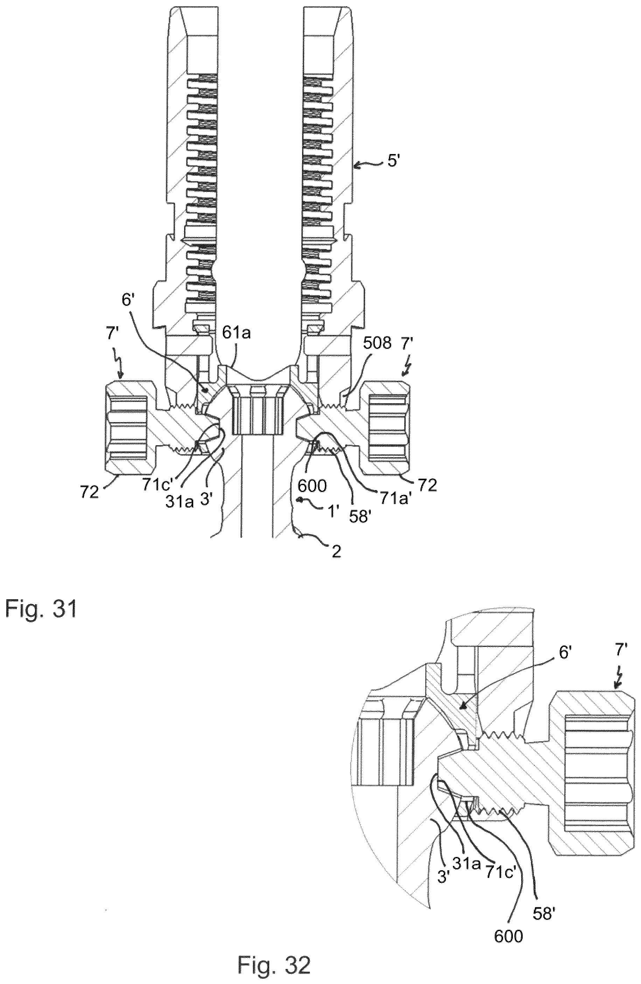

Referring now to Figs. 31 to 51, a third and a fourth embodiment of the bone anchoring device is described. The parts form a modular system and can be combined in various manners to provide a polyaxial bone anchoring device, a monoaxial bone anchoring device or a uniplanar bone anchoring device.

First, with respect to Figs. 31 to 45, the monoaxial bone anchoring device will be explained. The monoaxial bone anchoring device differs from the first embodiment according to Figs. 1 to 30 in the design of the receiving part, the head of the bone anchoring element and the restriction element. Referring specifically to Figs. 31 to 37, the receiving part 5' differs from the receiving part 5 in the position and shape of the threaded through holes 58' opening into the accommodation space 53 at a lower position. All other portions are identical or highly similar to that of the receiving part 5 of the first embodiment and the description thereof is not repeated. The transverse threaded through holes 58' are located at an axial position at a distance from the bottom end 5b of the receiving part 5' that corresponds to a position where an inserted head 3' has its greatest outer diameter. As can be seen in Figs. 31 and 32, the inserted restriction elements 7' engage the head 3' of the bone anchoring element 1 substantially at the region where the head 3' has its greatest outer diameter. Moreover, in the outer surface around the threaded through holes 58' a countersink 508 is formed that provides an abutment surface 508a for a restriction element of the fourth embodiment.

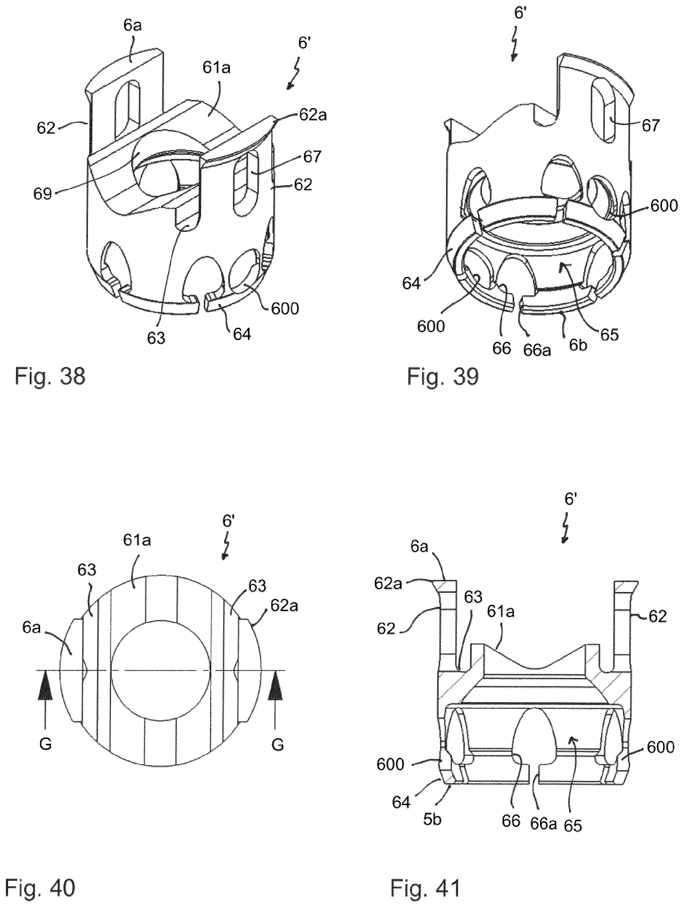

As shown in Figs. 38 to 41, the pressure element 6' differs from the pressure element 6 of the first embodiment in that it lacks the broadened lower portion of the elongate hole 67. Instead, the pressure element 6' has two through holes 600 that are located circumferentially at 180° offset from each other and below the elongate hole 67. The through holes 600 are unthreaded and serve for allowing a portion of the restriction element 7' to extend therethrough. When the pressure element 6' is in the receiving part 5' and in the pre-locking position in which the upper ring 62a is in the lower groove 57b, the through holes 600 are aligned with the threaded through holes 58' of the receiving part 5'.

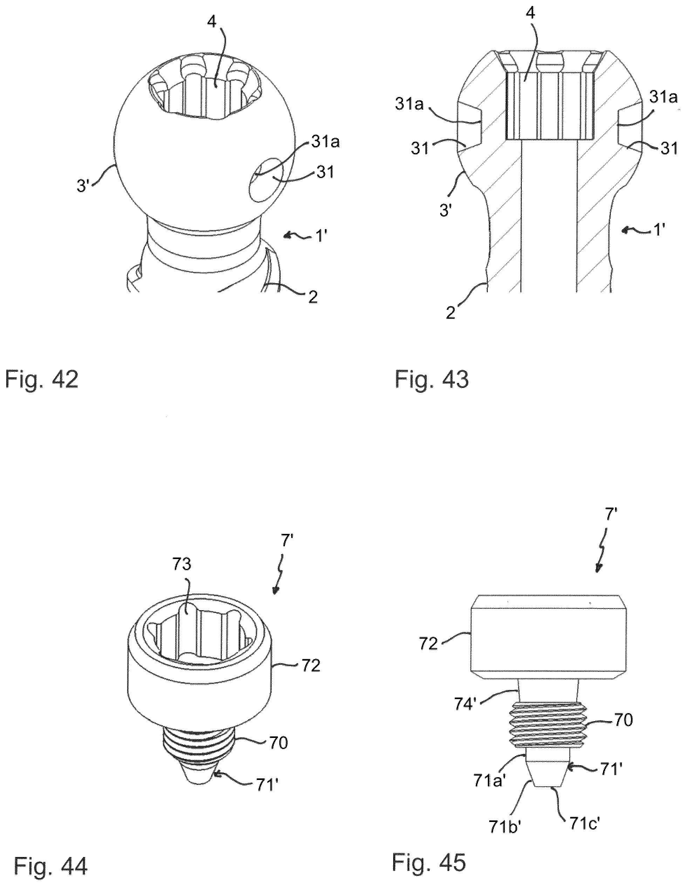

Referring now to Figs. 42 and 43, the bone anchoring element 1' will be explained. The bone anchoring element 1' differs from the bone anchoring element 1 of the first embodiment in the design of the head. The head 3' of the bone anchoring element 1' comprises at an axial position corresponding to the greatest diameter of the head 3' two recesses 31 that are in a circumferential direction 180° offset from each other. The recesses 31 are conically shaped corresponding to a front portion of the restriction element 7' and have a substantially flat bottom surface that forms a first cooperating surface 31a for cooperating with a second cooperating surface provided at the restriction element 7'. The dimension of the conical shape of the recesses 31 is such that a front portion of the restriction element 7' can be received therein with some play in a direction parallel to the central axis C when the bone anchoring device is assembled .

Optionally, the bone anchoring element 1' as well as the bone anchoring element 1 may have a coaxial channel 32 for introducing substances, such as bone cement to the bone.

Referring to Figs. 44 and 45, the restriction element 7' is explained. The restriction element 7' differs from the restriction element 7 of the first embodiment mainly in the design of the front portion. The restriction element 7' comprises the threaded portion 70 as I the first embodiment and an elongate front portion 71' that may have adjacent to the threaded portion 70 a cylindrical portion 71a' with an outer diameter smaller than that of the threaded portion 70 and slightly smaller than the diameter of the hole 600 in the pressure element 6'. Adjacent to the cylindrical portion 71a', there is a conical portion 71b' that narrows towards a flat end surface 71c' which is configured to cooperate with the bottom 31a of the recesses 31 of the head 3'. Thus, the flat end surface 71c' forms a second cooperating surface. The rear portion 72 may be identical to the rear portion of the restriction element 7 of the first embodiment. The predetermined breaking area 74' between the rear portion 72 and the threaded portion 70 may be conically widening between the threaded portion 70 and the rear portion 72. However, any other shape for the predetermined breaking area may be contemplated.

When the receiving part 5' with the pre-assembled pressure element 6' and the anchoring element 1' is used without the restriction elements 7', the bone anchoring device functions as a polyaxial bone anchoring device. If the clinical application requires a monoaxial bone anchoring device, the restriction elements 7' are screwed into the threaded transverse holes 58' until the flat end surface 71c' presses against the flat bottom surface 31a of the recesses in the head 3' of the bone anchoring element 1', respectively. By means of this, the shank 2 has a fixed orientation with respect to the receiving part 5' so that a monoaxial bone anchoring device is formed. Since the front portion 71' has an axial play in the hole 600 of the pressure element, when finally locking the bone anchoring device using the locking element 8, the locking force of the fixation element 8 is transferred to the head 3 via the pressure element 6'.

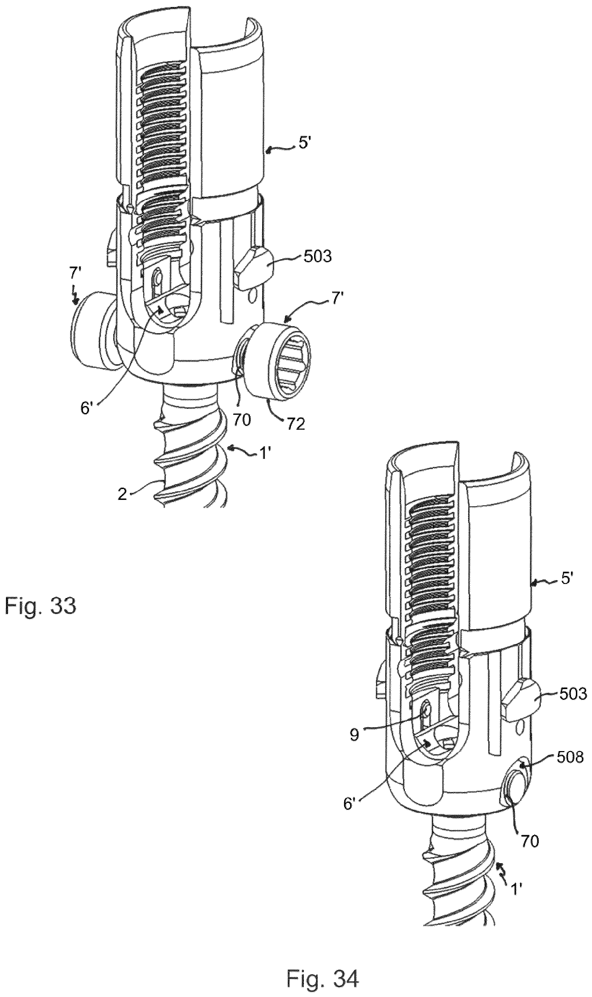

When the torque applied to the rear portion 72 exceeds a predetermined torque, the rear portion 72 is broken-off at the predetermined breaking area 74' as shown in Fig. 34.

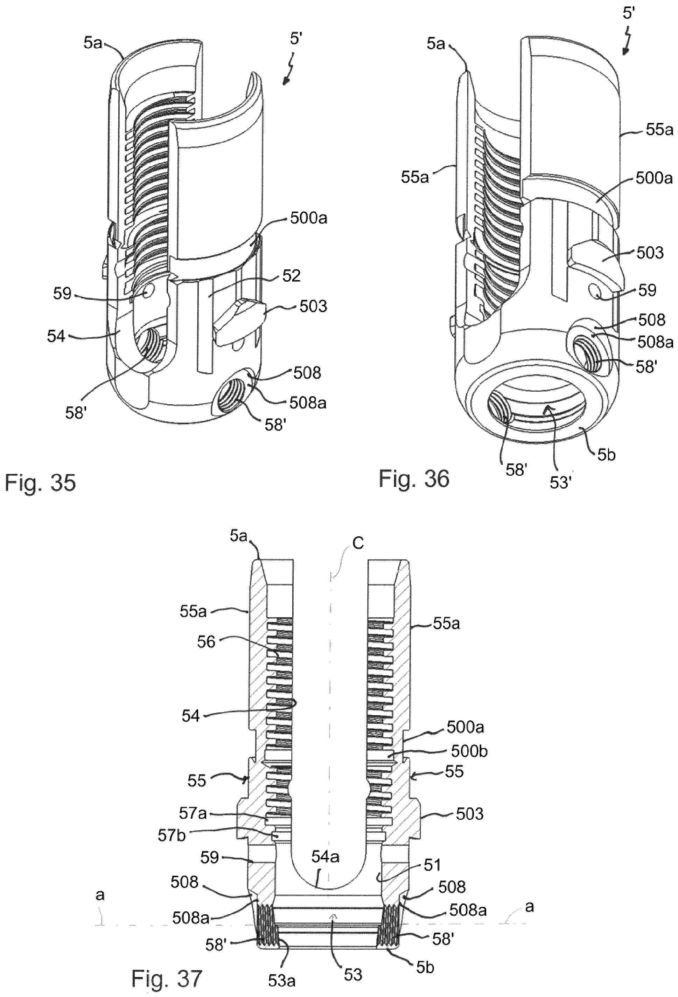

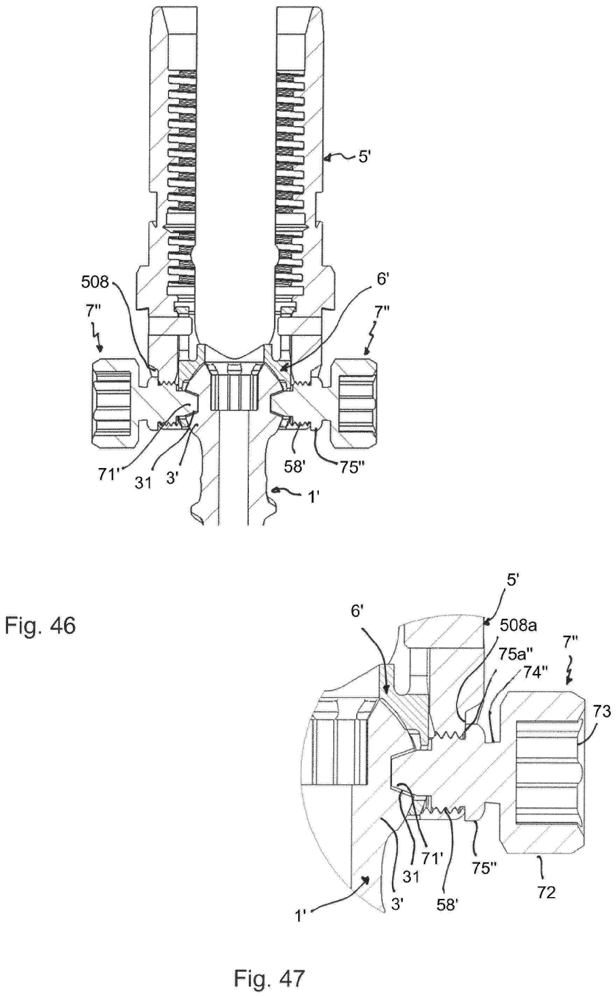

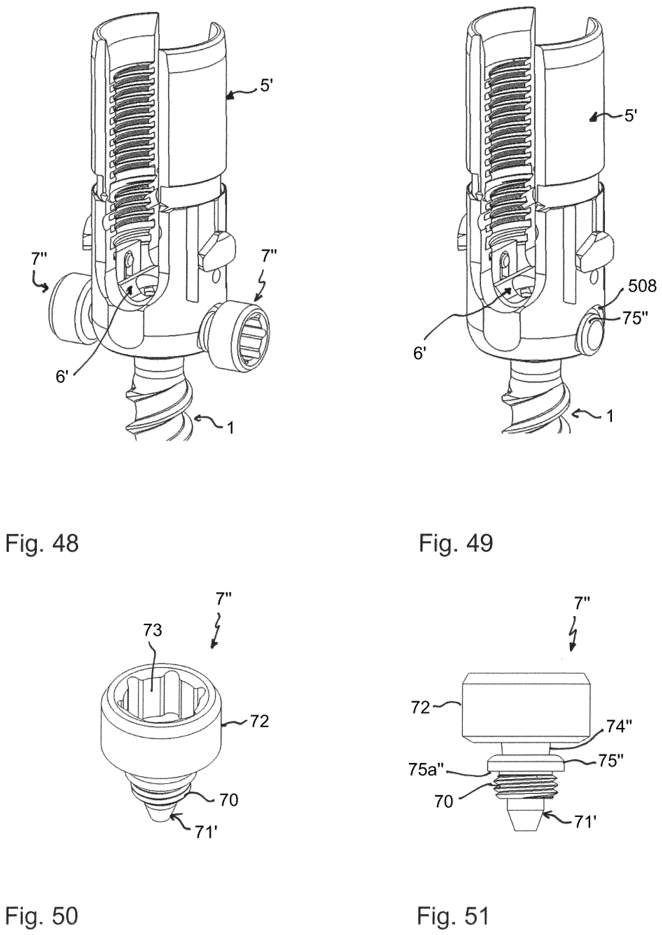

Referring to Figs. 46 to 51, the fourth embodiment of the bone anchoring device is described. The bone anchoring device of the fourth embodiment is identical to the bone anchoring device of the third embodiment except the design of the restriction elements. Hence, the receiving part 5', the pressure element 6' and the bone anchoring element 1' are identical. The restriction elements 7" as shown in Figs. 50 and 51 differs from the restriction elements 7' according to Figs. 44 and 45 only by the additional provision of an annular projection 75" that is formed adjacent to the threaded portion 70 in the direction to the rear portion 72. The annular projection 75" has an outer diameter greater than the outer diameter of the threaded portion 70 and smaller than the outer diameter of the rear portion 72. On the side facing towards the free end surface 71c' the annular projection 75" comprises a flat abutment surface 75a" that is configured to cooperate with the flat abutment surface 508a on the bottom of the countersink 508 around the threaded through holes 58' of the receiving part 5' which is shown in Figs. 35 to 37. Hence, the flat abutment surface 75a" forms a second cooperating surface. The flat abutment surface 75a" is at an axial position with respect to the flat end surface 71c' such that when the first cooperating surface 508a and the second cooperating surface 75a" are pressed against each other as shown in Figs. 46 and 47, the conical front portion 71b' does not press onto the bottom 31a of the conical recess 31 in the head 3'. As a result, the bone anchoring element 1' can pivot around the pivot axis provided by the screw axis of the restriction elements 7". As a result, pivoting of the bone anchoring element 1' is restricted to a single plane. In the embodiment shown in Figs. 46 to 49 the single plane is defined by the plane spanned by the longitudinal axis of the rod channel and the central axis C of the receiving part 5'.

When a predetermined torque is exceeded, the rear portion 72 is broken-off so that a uniplanar bone anchoring device as shown in Fig. 49 is formed that can be inserted into the bone. Also in this embodiment, the axial play allows to move the pressure element 6' slightly downward when the bone anchoring device is finally locked by tightening the locking element 8. It shall be noted that depending on the exact position of the abutment surface 75a" or the length of the front portion 71', a restriction element 7" may be provided which is configured to exert a pre-load on the head 3' when the front surface 71c' slightly touches the bottom 31a of the recess 31 so that a specific angular position in the single plane can be temporarily maintained by friction. This position may be changed by overcoming the frictional force.

It shall further be noted that the holes 600 in the pressure element 6' can be located at another position, for example, the holes can be aligned with the rod channel so that a pivot plane for the bone anchoring element 1' can be perpendicular to the longitudinal axis of the rod channel. It can also be envisaged to have more than two opposite holes 600 to provide more than a single pivot plane. For example, four holes can be provided where two holes are circumferentially at a position of the legs of the pressure element 6' and two additional holes are aligned with the rod channel. The receiving part 5' may have threaded through holes 58' at corresponding positions. The head 3' may also have more than the two recesses 31.

In a further modification, the head 3 may have at its free end surface one or more indication features (not shown in the figures) that indicate the circumferential position of the recesses 31.

This may facilitate the alignment of the recesses 31 with the threaded holes 58' of the receiving part 5' when the head 3' is in the receiving part 5'.

With the bone anchoring device according to the third and the fourth embodiment, a modular system can be provided which permits to assemble a variety of bone anchoring devices out of only a few parts.

Modifications of the above-described embodiments are conceivable. In particular, the shape of the parts is not limited to the detailed shape as shown in the figures. Deviations may be possible and encompassed by the disclosure. Although in the embodiments two threaded through holes are shown, only one threaded through hole and one restriction element may be sufficient.

Instead of the locking member 8 being a set screw all other kinds of locking assemblies known in the art may be used. For the bone anchoring element all types of bone anchoring elements that are suitable for anchoring in bone or a vertebra, such as bone screws, bone nails, etc. may be used. As used in the present specification and the appended claims, the term "rod" shall be understood as including any elongate member, regardless of the cross-sectional shape of the elongate member. Specifically, a spinal stabilization rod as used herein may have a substantially circular, oval, or angular cross-section. Such a cross-section may further vary along a length of the rod. The rod may be stiff or flexible.

Moreover, the accommodation space of the receiving part and the pressure element may have a design that allows to pivot the bone anchoring element to a greater pivot angle to one side compared to other sides.

## Claims

1. A bone anchoring device for coupling a rod to bone, including
an anchoring element (1, 1') comprising a shank (2) for anchoring in bone and a head (3, 3'),
a receiving part (5, 5') having a first end (5a) and a second end (5b), a channel (54) for receiving the rod (100), a passage (51) extending from the first end to the second end, the passage (51) defining a central axis (C), and an accommodation space (53) for accommodating the head (3, 3') of the bone anchoring element (1, 1'), and at least one through hole (58, 58') transverse to the central axis (C), the transverse through hole (58, 58') comprising an internal thread,
at least one restriction element (7, 7', 7") configured to be inserted into the through hole (58, 58') of the receiving part (5, 5'), the restriction element comprising an external thread;
a pressure element (6, 6') configured to be arranged in the passage (51) to exert pressure onto the head (3, 3') when the head (3, 3') and the pressure element (6, 6') are in the receiving part (5, 5') such that the head (3, 3') is pivotable with respect to the receiving part (5, 5') and can be locked at an angle relative to the receiving part (5, 5');
wherein when the restriction element (7, 7', 7") is in the through hole (58, 58') it is configured to interact with the pressure element (6, 6') or with the head (3, 3') to prevent a pivoting motion of the head (3, 3') in the receiving part (5, 5') or to restrict a pivoting motion of the head (3, 3') to a single plane.

2. The bone anchoring device of claim 1, wherein the pressure element (6, 6') is configured to encompass the head (3, 3') laterally and from a free end of the head (3, 3'), preferably wherein the pressure element is a monolithic part.

3. The bone anchoring device of claim 1 or 2, wherein a longitudinal axis (a) of the through hole (58, 58') is perpendicular to the central axis (C).

4. The bone anchoring device of one of claims 1 to 3, wherein the restriction element (7, 7', 7") comprises a portion (72) that is configured to be broken-off at a breaking area (74, 74', 74') once the restriction element (7, 7', 7") has engaged the pressure element (6, 6') or the head (3, 3') to prevent or restrict a pivoting motion of the head.

5. The bone anchoring device of one of claims 1 to 4, wherein two transverse through holes (58, 58') are provided at opposite sides from the central axis (C), preferably at circumferential positions perpendicular to a longitudinal axis of the channel for the rod.

6. The bone anchoring device of one of claims 1 to 5, wherein the pressure element (6) comprises a first cooperating surface (67a) that cooperates with a second cooperating surface 71) of the restriction element (7) in such a way that a force is exerted onto the head (3) that locks the head in the receiving part (5) such that pivoting of the head (3) is prevented.

7. The bone anchoring device of claim 6, wherein the first cooperating surface (67a) and the second cooperating surface (71) are inclined relative to the central axis (C).

8. The bone anchoring device of one of claims 1 to 7, wherein the receiving part is a first receiving part (5) configured to receive a first rod and wherein the bone anchoring device further comprises a second receiving part (150) for receiving a second rod, and wherein the second receiving part (150) is configured to be connected to the first receiving part (5) by means of a connection element (170) that engages the second receiving part (150) and the transverse through hole (58) of the first receiving part (5).

9. The bone anchoring device of claim 8, wherein the second receiving part (150) comprises a through hole (153) through which the connection element (170) extends into the transverse through hole (58) of the first receiving part (5), preferably wherein the connection element (170) comprises an abutment surface (175a) that abuts against a shoulder (152a) at an outer edge of the through hole (153) of the second receiving part (150).

10. The bone anchoring device of claim 8 or 9, wherein a channel (154) in the second receiving part (150) for receiving the second rod is parallel to the channel (54) in the first receiving part (5) for receiving the first rod.

11. The bone anchoring device of one of claims 1 to 10, wherein the head (3') comprises at least one recess (31), preferably two opposite recesses, extending transverse to the shank axis for engagement with the restriction element (7', 7").

12. The bone anchoring device of claim 11, wherein the recess (31) is located at an axial position substantially, preferably exactly, at a position where the head (3') has a greatest outer diameter.

13. The bone anchoring device of one of claims 11 or 12, wherein the restriction element (7') cooperates with the head (3') such that a pivoting motion of the head is prevented.

14. The bone anchoring device of one of claims 11 to 13, wherein the head (3') comprises a first cooperation surface (31a) and the restriction element (7') comprises a second cooperating surface (71c') and wherein the first and the second cooperating surfaces extend substantially parallel to the central axis (C), preferably wherein the first cooperating surface (31a) is a bottom of a recess (31) in the head (3') and the second cooperating surface (71c') is an end surface of the restriction element (7').

15. The bone anchoring device of one of claims 11 or 12, wherein the restriction element (7") cooperates with the head (3') such that the head (3') is configured to pivot in a single plane, preferably, wherein a first cooperating surface (508a) is formed at an edge of the transverse hole of the receiving part (5) and a second cooperating surface (75") is formed at the restriction element (7") such that a front portion (71') of the restriction element (7") is received in the recess (31) of the head (3') with a play to permit the head (3') to pivot in a single plane.

## Patentansprüche

1. Knochenverankerungsvorrichtung zum Verbinden eines Stabes mit einem Knochen, umfassend
ein Verankerungselement (1, 1'), das einen Schaft (2) zur Verankerung im Knochen und einen Kopf (3, 3') umfasst,
ein Aufnahmeteil (5, 5') mit einem ersten Ende (5a) und einem zweiten Ende (5b), einem Kanal (54) zur Aufnahme des Stabs (100), einem Durchgang (51), der sich vom ersten Ende zum zweiten Ende erstreckt, wobei der Durchgang (51) eine Mittelachse (C) definiert, und einem Aufnahmeraum (53) zur Aufnahme des Kopfes (3, 3') des Knochenverankerungselements (1, 1'), und zumindest einem Durchgangsloch (58, 58') quer zur Mittelachse (C), wobei das quer verlaufende Durchgangsloch (58, 58') ein Innengewinde umfasst,
zumindest ein Begrenzungselement (7, 7', 7"), das dazu ausgebildet ist, in das Durchgangsloch (58, 58') des Aufnahmeteils (5, 5') eingeführt zu sein, wobei das Begrenzungselement ein Außengewinde umfasst;
ein Druckelement (6, 6'), das dazu ausgebildet ist, in dem Durchgang (51) angeordnet zu sein, um Druck auf den Kopf (3, 3') auszuüben, wenn der Kopf (3, 3') und das Druckelement (6, 6') im Aufnahmeteil (5, 5') sind, so dass der Kopf (3, 3') relativ zum Aufnahmeteil (5, 5') schwenkbar ist und in einem Winkel relativ zum Aufnahmeteil (5, 5') verriegelt sein kann;
wobei das Begrenzungselement (7, 7', 7"), wenn es in dem Durchgangsloch (58, 58') ist, dazu ausgebildet ist, mit dem Druckelement (6, 6') oder mit dem Kopf (3, 3') zusammenzuwirken, um eine Schwenkbewegung des Kopfes (3, 3') im Aufnahmeteil (5, 5') zu verhindern oder eine Schwenkbewegung des Kopfes (3, 3') auf eine einzige Ebene zu beschränken.

2. Knochenverankerungsvorrichtung nach Anspruch 1, wobei das Druckelement (6, 6') dazu ausgebildet ist, den Kopf (3, 3') seitlich und von einem freien Ende des Kopfes (3, 3') her zu umschließen, wobei das Druckelement vorzugsweise ein einteiliges Element ist.

3. Knochenverankerungsvorrichtung nach Anspruch 1 oder 2, wobei eine Längsachse (a) des Durchgangslochs (58, 58') senkrecht zur Mittelachse (C) ist.

4. Knochenverankerungsvorrichtung nach einem der Ansprüche 1 bis 3, wobei das Begrenzungselement (7, 7', 7") einen Abschnitt (72) umfasst, der dazu ausgebildet ist, an einem Bruchbereich (74, 74', 74') abgebrochen zu werden, sobald das Begrenzungselement (7, 7', 7") mit dem Druckelement (6, 6') oder dem Kopf (3, 3') in Eingriff ist, um eine Schwenkbewegung des Kopfes zu verhindern oder zu begrenzen.

5. Knochenverankerungsvorrichtung nach einem der Ansprüche 1 bis 4, wobei zwei quer verlaufende Durchgangslöcher (58, 58') auf gegenüberliegenden Seiten der Mittelachse (C) vorgesehen sind, vorzugsweise an Umfangspositionen senkrecht zu einer Längsachse des Kanals für den Stab.

6. Knochenverankerungsvorrichtung nach einem der Ansprüche 1 bis 5, wobei das Druckelement (6) eine erste Zusammenwirkungsfläche (67a) umfasst, die mit einer zweiten Zusammenwirkungsfläche (71) des Begrenzungselements (7) derart zusammenwirkt, dass eine Kraft auf den Kopf (3) ausgeübt wird, die den Kopf im Aufnahmeteil (5) verriegelt, so dass ein Schwenken des Kopfes (3) verhindert ist.

7. Knochenverankerungsvorrichtung nach Anspruch 6, wobei die erste Zusammenwirkungsfläche (67a) und die zweite Zusammenwirkungsfläche (71) relativ zur Mittelachse (C) geneigt sind.

8. Knochenverankerungsvorrichtung nach einem der Ansprüche 1 bis 7, wobei das Aufnahmeteil ein erstes Aufnahmeteil (5) ist, das dazu ausgebildet ist, einen ersten Stab aufzunehmen, und wobei die Knochenverankerungsvorrichtung weiter ein zweites Aufnahmeteil (150) zur Aufnahme eines zweiten Stabs umfasst, und wobei das zweite Aufnahmeteil (150) dazu ausgebildet ist, mit dem ersten Aufnahmeteil (5) mittels eines Verbindungselements (170) verbunden zu sein, das mit dem zweiten Aufnahmeteil (150) und dem quer verlaufenden Durchgangsloch (58) des ersten Aufnahmeteils (5) in Eingriff ist.

9. Knochenverankerungsvorrichtung nach Anspruch 8, wobei das zweite Aufnahmeteil (150) ein Durchgangsloch (153) umfasst, durch das sich das Verbindungselement (170) in das quer verlaufende Durchgangsloch (58) des ersten Aufnahmeteils (5) erstreckt, wobei das Verbindungselement (170) vorzugsweise eine Anschlagsfläche (175a) umfasst, die an einer Schulter (152a) an einem äußeren Rand des Durchgangslochs (153) des zweiten Aufnahmeteils (150) anstößt.

10. Knochenverankerungsvorrichtung nach Anspruch 8 oder 9, wobei ein Kanal (154) im zweiten Aufnahmeteil (150) zur Aufnahme des zweiten Stabs parallel zu dem Kanal (54) im ersten Aufnahmeteil (5) zur Aufnahme des ersten Stabs verläuft.

11. Knochenverankerungsvorrichtung nach einem der Ansprüche 1 bis 10, wobei der Kopf (3') zumindest eine Ausnehmung (31), vorzugsweise zwei gegenüberliegende Ausnehmungen, umfasst, die sich quer zur Schaftachse erstrecken zum Eingriff mit dem Begrenzungselement (7', 7").

12. Knochenverankerungsvorrichtung nach Anspruch 11, wobei sich die Ausnehmung (31) an einer axialen Position befindet, die im Wesentlichen, vorzugsweise genau, an der Stelle liegt, an der der Kopf (3') seinen größten Außendurchmesser aufweist.

13. Knochenverankerungsvorrichtung nach einem der Ansprüche 11 oder 12, wobei das Begrenzungselement (7') mit dem Kopf (3') derart zusammenwirkt, dass eine Schwenkbewegung des Kopfes verhindert ist.

14. Knochenverankerungsvorrichtung nach einem der Ansprüche 11 bis 13, wobei der Kopf (3') eine erste Zusammenwirkungsfläche (31a) umfasst und das Begrenzungselement (7') eine zweite Zusammenwirkungsfläche (71c') aufweist und wobei sich die erste und die zweite Zusammenwirkungsfläche im Wesentlichen parallel zur Mittelachse (C) erstrecken, vorzugsweise wobei die erste Zusammenwirkungsfläche (31a) ein Boden einer Ausnehmung (31) im Kopf (3') ist und die zweite Zusammenwirkungsfläche (71c') eine Endfläche des Begrenzungselements (7') ist.

15. Knochenverankerungsvorrichtung nach einem der Ansprüche 11 oder 12, wobei das Begrenzungselement (7") mit dem Kopf (3') derart zusammenwirkt, dass der Kopf (3') dazu ausgebildet ist, in einer einzigen Ebene zu schwenken, vorzugsweise wobei eine erste Zusammenwirkungsfläche (508a) an einem Rand des Querlochs des Aufnahmeteils (5) ausgebildet ist und eine zweite Zusammenwirkungsfläche (75") am Begrenzungselement (7") ausgebildet ist, so dass ein vorderer Abschnitt (71') des Begrenzungselements (7") mit einem Spiel in der Ausnehmung (31) des Kopfes (3') aufgenommen ist, um dem Kopf (3') das Schwenken in einer einzigen Ebene zu ermöglichen.

## Revendications

1. Dispositif d'ancrage osseux pour coupler une tige à un os, comprenant
un élément d'ancrage (1, 1') comprenant un manche (2) pour l'ancrage dans l'os et une tête (3, 3'),
une partie de réception (5, 5') ayant une première extrémité (5a) et une seconde extrémité (5b), un canal (54) pour recevoir la tige (100), un passage (51) s'étendant de la première extrémité à la seconde extrémité, le passage (51) définissant un axe central (C), et un espace de logement (53) pour loger la tête (3, 3') de l'élément d'ancrage osseux (1, 1'), et au moins un trou traversant (58, 58') transversal à l'axe central (C), le trou traversant transversal (58, 58') comprenant un filetage interne,
au moins un élément de restriction (7, 7', 7") configuré pour être inséré dans le trou traversant (58, 58') de la partie de réception (5, 5'), l'élément de restriction comprenant un filetage externe ;
un élément de pression (6, 6') configuré pour être agencé dans le passage (51) pour exercer une pression sur la tête (3, 3') lorsque la tête (3, 3') et l'élément de pression (6, 6') sont dans la partie de réception (5, 5') de sorte que la tête (3, 3') peut pivoter par rapport à la partie de réception (5, 5') et peut être verrouillée à un angle par rapport à la partie de réception (5, 5') ;
dans lequel lorsque l'élément de restriction (7, 7', 7") est dans le trou traversant (58, 58'), il est configuré pour interagir avec l'élément de pression (6, 6') ou avec la tête (3, 3') pour empêcher un mouvement de pivotement de la tête (3, 3') dans la partie de réception (5, 5') ou pour restreindre un mouvement de pivotement de la tête (3, 3') à un seul plan.

2. Dispositif d'ancrage osseux selon la revendication 1, dans lequel l'élément de pression (6, 6') est configuré pour entourer la tête (3, 3') latéralement et à partir d'une extrémité libre de la tête (3, 3'), de préférence dans lequel l'élément de pression est une partie monolithique.

3. Dispositif d'ancrage osseux selon la revendication 1 ou 2, dans lequel un axe longitudinal (a) du trou traversant (58, 58') est perpendiculaire à l'axe central (C).

4. Dispositif d'ancrage osseux selon l'une des revendications 1 à 3, dans lequel l'élément de restriction (7, 7', 7") comprend une partie (72) qui est configurée pour être rompue au niveau d'une zone de rupture (74, 74', 74') une fois que l'élément de restriction (7, 7', 7") a mis en prise l'élément de pression (6, 6') ou la tête (3, 3') pour empêcher ou restreindre un mouvement de pivotement de la tête.

5. Dispositif d'ancrage osseux selon l'une des revendications 1 à 4, dans lequel deux trous traversants transversaux (58, 58') sont prévus au niveau de côtés opposés à partir de l'axe central (C), de préférence au niveau de positions circonférentielles perpendiculaires à un axe longitudinal du canal pour la tige.

6. Dispositif d'ancrage osseux selon l'une des revendications 1 à 5, dans lequel l'élément de pression (6) comprend une première surface de coopération (67a) qui coopère avec une seconde surface de coopération (71) de l'élément de restriction (7) de sorte qu'une force est exercée sur la tête (3) qui verrouille la tête dans la partie de réception (5) de sorte que le pivotement de la tête (3) est empêché.

7. Dispositif d'ancrage osseux selon la revendication 6, dans lequel la première surface de coopération (67a) et la seconde surface de coopération (71) sont inclinées par rapport à l'axe central (C).

8. Dispositif d'ancrage osseux selon l'une des revendications 1 à 7, dans lequel la partie de réception est une première partie de réception (5) configurée pour recevoir une première tige et dans lequel le dispositif d'ancrage osseux comprend en outre une seconde partie de réception (150) pour recevoir une seconde tige, et dans lequel la seconde partie de réception (150) est configurée pour être raccordée à la première partie de réception (5) au moyen d'un élément de raccordement (170) qui met en prise la seconde partie de réception (150) et le trou traversant transversal (58) de la première partie de réception (5).

9. Dispositif d'ancrage osseux selon la revendication 8, dans lequel la seconde partie de réception (150) comprend un trou traversant (153) à travers lequel l'élément de raccordement (170) s'étend dans le trou traversant transversal (58) de la première partie de réception (5), de préférence dans lequel l'élément de raccordement (170) comprend une surface de butée (175a) qui vient en butée contre un épaulement (152a) au niveau d'un bord externe du trou traversant (153) de la seconde partie de réception (150).

10. Dispositif d'ancrage osseux selon la revendication 8 ou 9, dans lequel un canal (154) dans la seconde partie de réception (150) pour recevoir la seconde tige est parallèle au canal (54) dans la première partie de réception (5) pour recevoir la première tige.

11. Dispositif d'ancrage osseux selon l'une des revendications 1 à 10, dans lequel la tête (3') comprend au moins un évidement (31), de préférence deux évidements opposés, s'étendant transversalement à l'axe du manche pour la mise en prise avec l'élément de restriction (7', 7").

12. Dispositif d'ancrage osseux selon la revendication 11, dans lequel l'évidement (31) est situé au niveau d'une position axiale sensiblement, de préférence exactement, au niveau d'une position où la tête (3') a un diamètre externe le plus grand.

13. Dispositif d'ancrage osseux selon l'une des revendications 11 ou 12, dans lequel l'élément de restriction (7') coopère avec la tête (3') de sorte qu'un mouvement de pivotement de la tête est empêché.

14. Dispositif d'ancrage osseux selon l'une des revendications 11 à 13, dans lequel la tête (3') comprend une première surface de coopération (31a) et l'élément de restriction (7') comprend une seconde surface de coopération (71c') et dans lequel les première et seconde surfaces de coopération s'étendent sensiblement parallèlement à l'axe central (C), de préférence dans lequel la première surface de coopération (31a) est un fond d'un évidement (31) dans la tête (3') et la seconde surface de coopération (71c') est une surface d'extrémité de l'élément de restriction (7').

15. Dispositif d'ancrage osseux selon l'une des revendications 11 ou 12, dans lequel l'élément de restriction (7") coopère avec la tête (3') de sorte que la tête (3') est configurée pour pivoter dans un seul plan, de préférence, dans lequel une première surface de coopération (508a) est formée au niveau d'un bord du trou transversal de la partie de réception (5) et une seconde surface de coopération (75") est formée au niveau de l'élément de restriction (7") de sorte qu'une partie avant (71') de l'élément de restriction (7") est reçue dans l'évidement (31) de la tête (3') avec un jeu pour permettre à la tête (3') de pivoter dans un seul plan.
